Europäisches Patentamt

(19) European Patent Office

Office européen des brevets

(11) EP 0 723 020 A1

(12) EUROPEAN PATENT APPLICATION

(43) Date of publication:
24.07.1996 Bulletin 1996/30

(51) Int. Cl.$^6$: C12Q 1/42, C12Q 1/54, G01N 27/327

(21) Application number: 95202634.2

(22) Date of filing: 30.09.1995

(84) Designated Contracting States:
AT BE CH DE DK ES FR GB GR IE IT LI LU MC NL PT SE

(30) Priority: 23.01.1995 IT MI950109

(71) Applicant: BARILLA G. e R. F.lli - Società per Azioni
I-43100 Parma (IT)

(72) Inventors:
• Azzoni, Alfredo
I-43100 Parma (IT)

• Cavalieri, Barbara
I-43100 Parma (IT)
• Mazzei, Franco
I-00176 Roma (IT)
• Botre, Claudio
I-00100 Roma (IT)

(74) Representative: Perani, Aurelio et al
c/o JACOBACCI & PERANI S.p.A
Via Visconti di Modrone, 7
20122 Milano (IT)

(54) A method for the determination of organophosphoric pesticides in materials of vegetable origin

(57) A method is described for the detection and determination of organophosphoric pesticides in vegetable materials which may contain them, comprising the reaction of glucose-6-phosphate with acid phosphatase and glucose oxidase in a buffered aqueous medium with the formation of oxygenated water, the addition of an extract of the vegetable materials, with reversible inhibition of the acid phosphatase by the organophosphoric pesticides if present, and the measurement of any reduction in the rate of formation of oxygenated water due to the said inhibition of the acid phosphatase by means of an amperometric electrode.

A biosensor usable for carrying out the said method is also described.

FIG.2

EP 0 723 020 A1

## Description

The present invention relates generally to the detection of organic pesticides in materials of vegetable origin.

More particularly, the present invention relates to a method for the detection and determination of organophosphoric pesticides in materials of vegetable origin, of industrial concern in food manufacturing and agricultural fields.

It is known that in intensive cultivation of industrial products such as, for example, cereals, tobacco, plants from which edible oils are derived (soya, rape, sunflowers, etc), very large quantities of pesticides are used to protect the crops from attack by insects and parasites in general.

Among the various pesticides available, organophosphoric pesticides are very frequently used, these having a particularly strong activity due to their capacity to inhibit irreversibly the enzyme acetylcholinesterase in the insects and consequently to cause their deaths. The activity of organophosphoric pesticides is not however limited to inhibiting acetylcholinesterase in insects but also acts against other living organisms which contain acetylcholinesterase.

Thus, for example, signs of toxicity may occur in man following exposure to organophosphoric insecticides by inhalation, ingestion or contact, these signs being characterised by neurovegetative effects such as nausea, gastro-intestinal cramps, bronchial contractions and hyper secretion, arteriol hypertension and effects on the central nervous system such as tremors, convulsions and respiratory paralysis.

The checking of all materials of vegetable origin intended as a human food material, or at least for consumption and use by man, for the possible presence of organophosphoric pesticides above a predetermined safety threshold is therefore of fundamental importance.

Such checking has been carried out up till now mainly by chemical analysis with the aid of instruments, for example by gas chromatography, possibly coupled with mass spectrometry and HPLC liquid chromatography. Applications of these methods are given, for example, in the following publications: Scheichter, M. S., Hornstein, I., Adv. Pest. Control. Res. 1 (1957), 353; Sherma, J., Zweig, G., Anal. Chem., 55 (1983) 57R; Stein, K., Schewdt, G., Anal. Chim. Acta, 272 (1991) 73; Sherma, J., Anal. Chem., 65 (1993) 40R.

These methods enable detection to be carried out very accurately and are highly sensitive but have the disadvantage of requiring very expensive apparatus and of having to be carried out by highly specialised personnel. Moreover, their use is necessarily confined to chemical laboratories and it is difficult to envisage their use at the sampling site. All this leads to very high operating costs while limiting the checking necessarily to a very small number of samples.

Finally, the time needed to carry out an analysis by the methods indicated above is fairly long, generally of the order of several hours, since laborious preparation and pretreatment of the samples is necessary.

These methods are not therefore very well suited to use "in the field", for example by people in agricultural or manufacturing industries which require rapid analytical safety checks on batches of primary materials of vegetable origin from a toxicological point of view.

There is thus a very great need for analytical methods for determining organophosphoric compounds which are quicker and more economical to carry out than the instrumental methods of chemical analysis mentioned above and which, in particular, can be used in the field even by personnel who are not particularly specialised.

This requirement has been partly satisfied in the prior art by the provision of analytical methods based on the use of specific biosensors which are able to convert into electrically measurable signals, variations in the concentration of a product obtained from a reaction catalysed by an enzyme which is inhibited irreversibly by organophosphoric compounds.

Such analytical methods are based on the inhibition of the enzyme acetylcholinesterase by organophosphoric insecticides. Examples of such methods are given in the following publications: Guilbault, G.G., Kramer, D.N. Anal. Chem. 37 (1965) 1675; Tran-Minh, C. Ion Selective Electrode Rev. 7 (1985) 41; Marty, J.L., Sode, K., Karube, I, Anal. Chim. Acta, 228 (1989) 49; Bernabei, M., Cremisini, C., Mascini, M., Palleschi, G. Anal. Lett. 24 (8) (1991) 1317; Botrè C., Botrè F., Lorenti, G., Mazzei, F., Porcelli, F., Scibona, G., Simonetti, G. Sensors and Actuators B, 19, (1994) 689.

These methods do not require very expensive apparatus and can be carried out easily even in the field. However since these methods are based on an irreversible reaction between the compound analysed and a biosensor, the number of analyses which can be carried out with each biosensor is rather small (from 10 to 20) and, at the same time, the time required for the analyses is still rather high (about 30 minutes per sample).

The problem at the root of the present invention is that of providing a method for the detection and determination of organophosporic pesticides in materials of vegetable origin which may contain them, which is quicker and more economical to carry out than the instrumental methods of chemical analysis mentioned above, which may be used in the field even by personnel who are not particularly specialised and which can be repeated theoretically a limitless number of times.

This problem is solved by a method which includes the steps of:

- reacting together glucose-6-phosphate in concentrations of from $10^{-5}$ to $10^{-3}$ M and an enzyme system constituted by acid phosphatase (FA) and glucose oxidase (GOD) in a buffered aqueous medium, with the formation of oxygenated water,

- measuring the concentration of oxygenated water continuously by means of an amperometric electrode which is selective therefor,
- causing reversible inhibition of the acid phosphatase (FA) by organophosporic pesticides, if present, by the addition to the buffered aqueous medium of solutions obtained by solvent extraction of the vegetable materials,
- measuring any reduction in the rate of formation of oxygenated water by means of the amperometric electrode.

Preferably the enzymes F A and GOD are immobilised on at least one suitable support.

Conveniently these enzymes may be immobilised on a single support constituted by a membrane.

In a preferred embodiment of the method of the invention, the amperometric electrode used for the measurement is separated from the buffered aqueous medium by a first membrane of cellulose acetate in direct contact with the electrode itself and on the outside of which is superimposed at least a second membrane carrying the immobilised enzymes.

Suitably, the enzymes are immobilised each on one of the said at least a second membrane.

A further membrane constituted by a dialysis membrane is preferably superposed on the outside of the at least a second membrane.

The enzyme FA is preferably immobilised on one of the said at least a second membrane which is constituted by a cellulose acetate membrane.

The enzyme GOD is preferably immobilised on one of the said at least a second membrane which is constituted by a NYLON 6.6 membrane carrying carboxyl groups on its surface.

To advantage, the solutions made by extraction of vegetable materials are placed in contact first with the immobilised FA and then with the immobilised GOD.

In this case the amperometric electrode is separated from the buffered aqueous medium by means of a first membrane of cellulose acetate in direct contact with the electrode itself and on the outside of which there is superposed a second membrane constituted by the said NYLON 6.6 membrane carrying immobilised GOD and a third membrane of cellulose acetate, while the FA is immobilised on a bioreactor constituted by a column containing a suitable support, such as, for example, a polydextran like e.g. Sephadex, functionalised glass balls or hollow polysulphanate fibres.

The solvents which may be used for the extraction of the pesticides from the vegetable materials include water and polar solvents miscible with water.

The pH of the buffered aqueous medium is preferably between 4.0 and 7.0.

The amperometric electrodes coupled to the membranes carrying the enzyme systems belong to the class of "biosensors".

The method of the invention is based on the surprising experimental finding that organophosphoric pesticides are powerful reversible inhibitors of the enzyme acid phosphatase which catalyses the following hydrolytic reaction of glucose-6-phosphate:

$$\text{glucose-6-phosphate} + H_2O \text{ ------> glucose} + H_2PO_4$$

The coupling of this hydrolysis reaction with a subsequent oxidation reaction catalysed by the enzyme glucose oxidase gives the following:

$$\text{glucose} + O_2 \xrightarrow{\text{GOD}} \text{gluconolactone} + H_2O_2$$

Gluconolactone and oxygenated water are obtained as the final products.

Oxygenated water is a chemical species which can easily be detected and determined quantitatively by means of an amperometric electrode which is selectively sensitive thereto.

In addition to parameters of an electrochemical nature, the selectivity of this electrode is also due to the presence of a cellulose acetate membrane which separates it from the aqueous medium with which it is in contact and which prevents the passage of compounds with a molecular weight higher than 100-150, which could interfere, such as for example ascorbic acid, uric acid etc.

The reactions which occur at the electrodes are as follows:

$$H_2O_2 \text{ ------> } O_2 + 2H^+ + 2e$$

$$1/2O_2 + 2H^+ + 2e \text{ ------> } H_2O$$

The organophosphoric pesticides have considerable inhibiting power towards FA and consequently slow the hydrolysis of the glucose-6-phosphate noticeably, in a dose-dependant manner, reducing production of glucose.

The smaller quantity of glucose produced in the primary reaction in turn causes the formation of a smaller quantity of oxygenated water which is readily detected and determined by the specific amperometric electrode.

At this point, after suitable preliminary calibrations, it is possible to determine the concentration of the organophosphoric pesticides on the basis of the concentration of oxygenated water detected by the electrode.

It is worth stressing that the inhibition of the enzyme acid phosphatase by the molecules of organophosphoric pesticides is of reversible type which allows the method of the invention to be repeated an indefinite number of times, in which it differs from the prior art enzymatic methods described above which are based on irreversible inhibitions of enzymes.

The method according to the invention will now be explained in greater detail with reference to the appended drawings, in which:

Figure 1 is a schematic illustration of the amperometric electrode of the electrochemical biosensor;
Figure 2 is a schematic illustration of the electrochemical biosensor;
Figure 3 shows schematically apparatus for carrying out the method of the invention;
Figure 4 illustrates schematically a variant of the apparatus for carrying out the method of the invention.

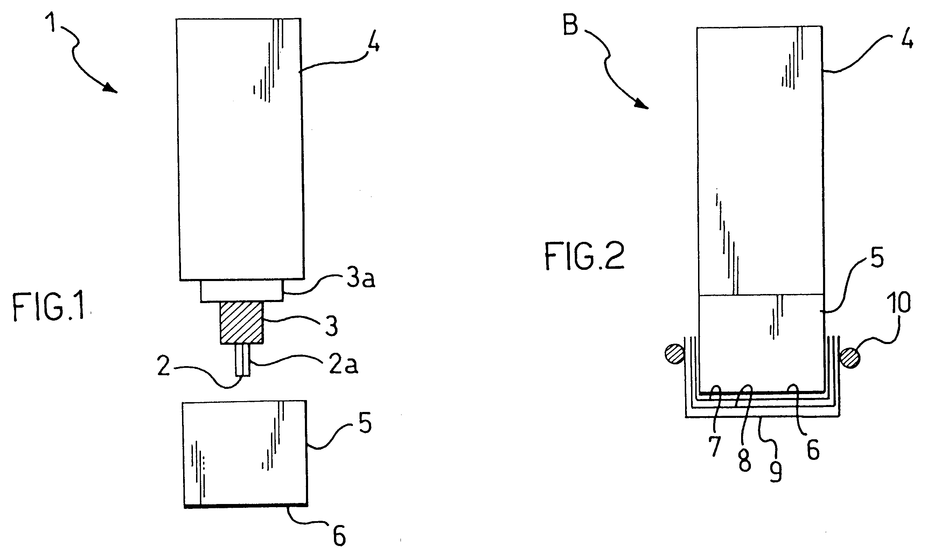

With reference to Figure 1, an amperometric electrode for carrying out the method of the invention includes a commercial sensor (1) for sensing oxygenated water constituted, for example, by a platinum wire electrode (2) coated with a layer (2a) of an epoxy resin which separates it and insulates it from an Ag/AgCl reference electrode (3) disposed coaxially around it. The electrodes (2) and (3) are fixed in a cylindrical container (4) filled with an electrolytic solution or an electrolytic gel and with the interposition of a suitable cover (3a). One end of the platinum electrode (2) is, however, bare so that it can act as the indicator electrode for the oxygenated water in the buffered aqueous medium. This electrode (2) is kept at a potential of about +650 mV relative to the reference electrode (3). A cap (5) which is also cylindrical is screwed on to that end of the cylindrical container (4) from which the bare end of the platinum electrode (2) projects. The end face of the cylindrical cap (5) is constituted by a cellulose acetate membrane (6) which is permeable to oxygenated water.

With reference to Figure 2, layers are provided on the membrane (6) of a biosensor (B) according to the invention, in the order, a membrane (7) on which glucose oxidase (GOD) is immobilised, a membrane (8) on which acid phosphatase (FA) is immobilised and a dialysis membrane (9). The membranes (7), (8) and (9) are kept firmly in position by means of an split ring (10).

It should be noted that the enzymes GOD and FA may alternatively be immobilised on the same membrane (7); in this case there will be no membrane indicated (8) in Figure 2.

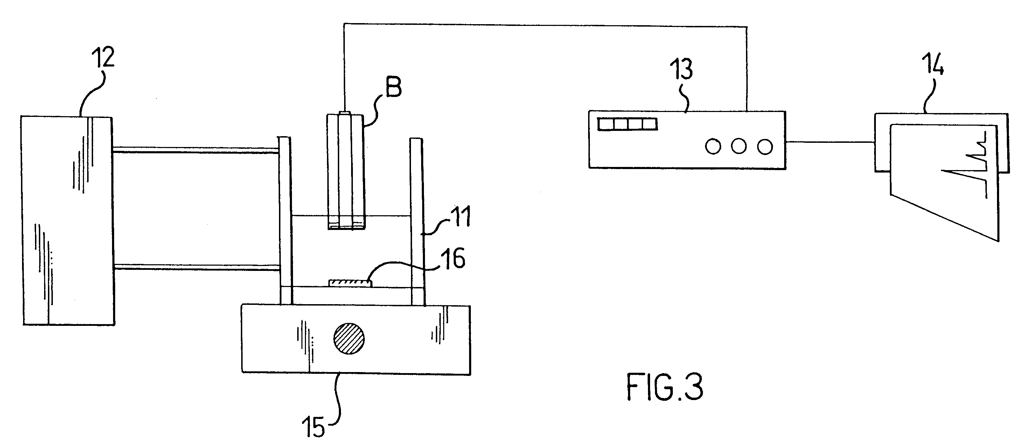

With reference to Figure 3, the biosensor (B) is inserted in a cell (11) kept at a constant temperature by a thermostat (12) and containing an aqueous solution buffered to a pH of between 4 and 7 in which the biosensor (B) is partially immersed; this latter is connected to an amperometric meter (13) having an output connected to a graphic recorder (14) or to any other data acquisition system.

The cell (11) is conveniently located on a magnetic stirrer (15) provided with a stirring element (16).

The method of the invention will be further described with reference to one embodiment provided purely by way of non-limitative example.

**EXAMPLE**

A sample of wheat flour was tested for any organophosphoric pesticide content by the method of the invention with the use of the apparatus shown schematically in Figure 3.

The biosensor membrane (7) containing immobilised GOD was prepared in the following manner.

Glucose oxidase (150000 U/g of solid from Aspergillus niger) was dissolved in 20 ml of polyazetidine prepolymer (PAP) and placed in contact with a NYLON 6.6 membrane, with a pore size of 0.2 mm and carboxyl groups on its surface, for 24 hours at ambient temperature.

After this period of time, the membrane was washed with 0.1 M citrate buffer with pH 6.0.

The biosensor membrane (8) containing immobilised FA was prepared as follows.

IV-S acid phosphatase (5 U/mg from Solanum tuberosum) was dissolved in 20 ml of PAP, placed in contact with a cellulose acetate membrane with a thickness of 0.0254 mm and MW cut = 12000 D (dialysis membrane), for 24 hours at ambient temperature and finally washed with citrate buffer (0.1 M, pH 6.0).

The biosensor including the said membranes was immersed in the thermostatted cell (11) described above containing 5.0 ml of 0.1 M citrate buffer at pH 6.0 and $10^{-4}$M glucose-6-phosphate kept at 30°C and connected to the amperometric meter (13).

A 2.0 g sample of wheat flour was extracted with 20.0 ml of 0.1 M citrate buffer at pH 6.0.

The aqueous extract was concentrated by centrifugation-filtration; 0.5 ml of concentrated extract was added to the buffered solution in the thermostated cell (11) and kept under agitation by means of the magnetic stirrer (15).

The variation in the current strength which occurred as a result of the addition of the extract to be tested was proportional to the reduction in the rate of formation of the oxygenated water, which was in turn proportional to the organophosphoric pesticide content of the solution added.

This content could be quantified from a calibrated curve obtained previously from known concentrations of an organophosphoric pesticide, paraoxon, between 3 and 30 parts per thousand million(ppb).

In the case in question, 8+/-1 ppb of organophosphoric compounds were found in wheat flour.

A sample of this same flour was subjected to analysis by gas chromatography, in accordance with one of the methods proposed by J.Sherma, J.Anal.Chem., 65 (1993) 40R, to determine its organophosphoric compound content.

The results of this analysis gave the organophosphoric compound content as 7.7+/- 0.2 ppb.

This confirms the reliability of the method of the present invention.

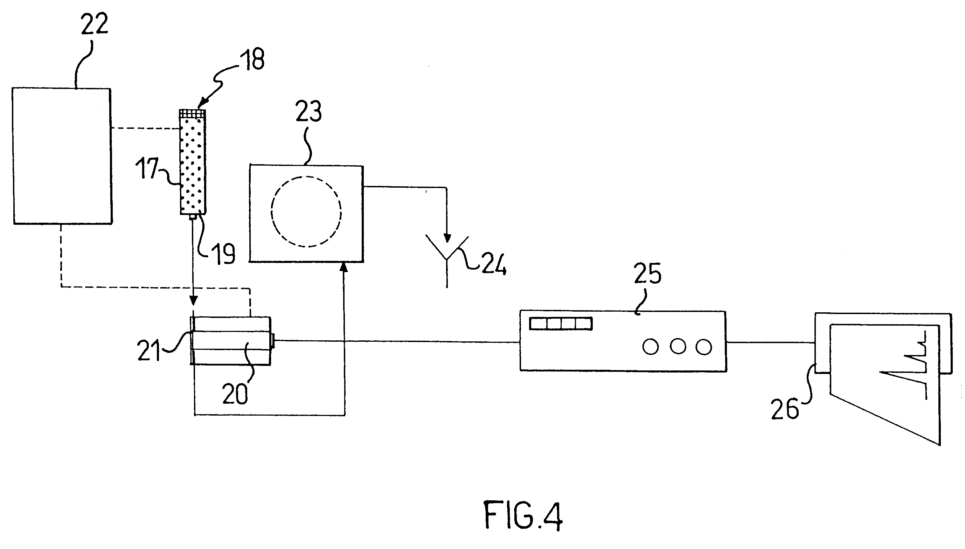

With reference to Figure 4, an alternative piece of equipment for carrying out the method of the invention comprises a bioreactor (17) constituted essentially by a column containing immobilised FA, provided with an aperture (18) for the inlet of fluid to be analysed and an opposite opening (19) for the outlet thereof, a biosensor, (20) identical to the biosensor (B) described above except for the absence of the membrane (8) carrying immobilised FA, inserted in a flow cell (21) kept at a constant temperature by a thermostat (22), and in fluid contact with the bioreactor (17) and with a peristaltic pump (23) connected to a discharge (24) for the fluid which has been measured. The electrode of the biosensor (20) is connected to an amperometric meter (25) the output of which is connected to a graphic recorder (26).

The bioreactor (17) may be formed, for example, by utilizing glass balls with a controlled porosity (180-260 Angstrom, 60-200 mesh), previously alkylaminated with 3-amino-propyl-triethoxy-silane; the amino groups which are available on the surface of the glass balls as a result of the alkylamination reaction are activated by glutaraldehyde (5% v/v in 0.1 M sodium bicarbonate solution). The glass balls thus activated are packed in a column through which a solution of the FA (500-1000 U) in a suitable buffer is passed at a flow rate of about 1.0 ml/min, with continuous recycling for about 4-5 hours at ambient temperature.

In an alternative method, the bioreactor (17) may be made by immobilising the FA physically on hollow polysulphonate fibres.

In an alternative embodiment of the invention, the method may be carried out with the use of the apparatus explained above with reference to Figure 4.

In this method, the concentrated aqueous extract obtained from a sample to be analysed is supplied to the bioreactor (17) through the inlet aperture (18). The bioreactor (17) is kept at a constant temperature by means of a thermostat (22) and the hydrolysis of the glucose-6-phosphate catalysed by the acid phosphatase immobilised on the support occurs in the column of the bioreactor (17).

The fluid leaving the bioreactor (17) is directed to the flow cell (21) which is also kept at a constant temperature by means of the thermostat (22) and which houses the biosensor (20) containing immobilised GOD. This latter catalyses the oxidation of the glucose as described above with the concomitant production of oxygenated water which is detected by the biosensor (20) with the production of an electrical signal which is sent to the amperometric meter (25) and recorded by means of the graphic recorder (26).

The fluid leaving the flow cell (21) is discharged by means of the peristaltic pump (23) through the discharge opening (24).

The use of the method of the invention described above enables the analytical method to be automated with a consequent reduction in the analysis times and a simplification of the procedure.

A simplified version of the method just described provides for the replacement of the bioreactor (17) with FA and the biosensor (20) with GOD by a bioenzymatic biosensor identical to the biosensor (B) described above but incorporated in the thermostatted flow cell (21).

It is interesting to note that vegetable tissues containing the enzymes can conveniently be used instead of the purified enzymes described above. For example thin sections of Solanum tuberosum, that is, ordinary potatoes, may be used instead of acid phosphatase, with obvious advantages from an economical point of view.

The perfect reproducibility of the method and its considerable accuracy, sensitivity, and simplicity of execution make it suitable for various applications in the food field (cereals, legumes, plants and seeds which are sources of edible oils, aromatic plants such as tea, coffee etc) and in the animal field (fodder). More particularly, the method of the invention lends itself to the effective determination of any organophosphoric pesticides in tobacco, the presence of which is a serious toxicological risk bearing in mind that the way in which these substances are taken in (inhalation) causes their practically instantaneous and total absorption.

Finally it is noted that the method of the invention may easily be carried out even outside a chemical laboratory and even by personnel who are not particularly specialised, at little cost overall.

## Claims

1. A method for the detection and determination of organophosphoric pesticides in materials of vegetable origin which may contain them, comprising the steps of:

- reacting together glucose-6-phosphate in concentrations of from $10^{-5}$ to $10^{-3}$ M and an enzyme system constituted by acid phosphatase (FA) and glucose oxidase (GOD) in a buffered aqueous medium, with the formation of oxygenated water,
- measuring the concentration of oxygenated water continuously by means of an amperometric electrode which is selective therefor,
- causing reversible inhibition of the acid phosphatase (FA) by organophosporic pesticides, if present, by the addition to the buffered aqueous medium of solutions obtained by solvent extraction of the said materials of vegetable origin,
- measuring any reduction in the rate of formation of oxygenated water by means of the amperometric electrode.

2. A method according to Claim 1, characterised in that the FA and GOD are immobilised on at least one suitable support.

3. A method according to Claim 2, characterised in that the FA and GOD are immobilised on a single support constituted by a membrane.

4. A method according to Claim 2, characterised in that the amperometric electrode is separated from the buffered aqueous medium by a first membrane of cellulose acetate in direct contact with the electrode itself, at least a second membrane carrying the immobilised enzymes being superposed on the outside thereof.

5. A method according to Claim 4, characterised in that the enzymes are each immobilised on one of the said at least a second membrane.

6. A method according to any one of Claims 4 and 5, characterised in that a further membrane constituted by a dialysis membrane is superposed on the outside of the said at least a second membrane.

7. A method according to any one of Claims 4 and 6, characterised in that one of the said at least a second membrane is constituted by a cellulose acetate membrane on which FA is immobilised.

8. A method according to any one of Claims 4 and 6, characterised in that one of the said at least a second membrane is constituted by a NYLON 6.6 membrane carrying carboxyl groups on its surface on which the GOD is immobilised.

9. A method according to any one of the preceding claims, characterised in that the amperometric electrode is constituted by a platinum wire electrode (2) coated with a layer (2a) of an epoxy resin which separates and insulates it from a (Ag/AgCl) reference electrode (3) disposed coaxially around it, the two electrodes (2) and (3) being fixed in a cylindrical container (4) filled with an electrolytic solution or an electrolytic gel with the interposition of a suitable cover (3a), and in that the platinum electrode (2) acts as an indicator electrode for the oxygenated water.

10. A method according to Claim 9, characterised in that the platinum electrode (2) is kept at a potential of about +650 mV relative to the reference electrode (3).

11. A method according to Claim 10, characterised in that the amperometric electrode is separated from the buffered aqueous medium by a first membrane of cellulose acetate, on the outside of which are superposed a second membrane constituted by the NYLON 6.6 membrane carrying immobilised GOD and a third membrane of cellulose acetate.

12. A method according to Claim 11, characterised in that the FA is immobilised on an inert substrate in a column.

13. A method according to Claim 12, characterised in that the inert substrate is selected from the group comprising polydextran, functionalised glass balls and hollow polysulphonate fibres.

14. A biosensor (B) for detecting organophosphoric pesticides in materials of vegetable origin comprising a sensor (1) for sensing oxygenated water, including a cellulose acetate membrane (6) which is permeable to the oxygenated water, characterised in that the following layers are arranged on the membrane (6) in order: a first membrane (7) on which glucose oxidase is immobilised; a second membrane (8) of cellulose acetate on which acid phosphatase is immobilised; and a dialysis membrane (9), the membranes being held firmly in position by means of a split ring (10).

15. A biosensor (20) for detecting organophosphoric pesticides in materials of vegetable origin comprising a sensor (1) for sensing oxygenated water, including a cellulose acetate membrane (6) which is permeable to the oxygenated water, characterised in that the following layers are arranged on the membrane (6) in order: a membrane (7) on which GOD and FA are immobilised; and a dialysis membrane (9), the membranes being kept firmly in position by means of a split ring (10).

16. A biosensor (B) according to Claim 14, characterised in that the membrane (7) is of NYLON 6.6.

17. A biosensor (20) according to Claim 15, characterised in that the membrane (7) is of NYLON 6.6.

FIG.1

FIG.2

EP 0 723 020 A1

FIG.3

EP 0 723 020 A1

EP 0 723 020 A1

FIG.4

European Patent Office

## EUROPEAN SEARCH REPORT

**Application Number**

| | DOCUMENTS CONSIDERED TO BE RELEVANT | | EP 95202634.2 |
|---|---|---|---|
| Category | Citation of document with indication, where appropriate, of relevant passages | Relevant to claim | CLASSIFICATION OF THE APPLICATION (Int. Cl. 6) |
| A | CHEMICAL ABSTRACTS, vol. 114, no. 14, 1991, April 8, Columbus, Ohio, USA L. CAMPANELLA et al. "Determination of inorganic phosphate in drug formulations and biological fluids using a plant tissue electrode" page 436, no. 129 285y; & J. Pharm. Biomed. Anal. 1990, 8(8-12), 711-16 -- | 1,14 | C 12 Q 1/42<br>C 12 Q 1/54<br>G 01 N 27/327 |
| A | CHEMICAL ABSTRACTS, vol. 105, no. 21, 1986, November 24, Columbus, Ohio, USA A.A. KOMEIL "Insecticidal inhibition of the soil acid phosphatase" page 254, no. 185 847j; & Beitr. Trop. Landwirtsch. Veterinärmed. 1986, 24(2), 195-200 -- | 1 | |
| A | CHEMICAL ABSTRACTS, vol. 102, no. 3, 1985, January 21, Columbus, Ohio, USA AGENCY OF INDUSTRIAL SCIENCES AND TECHNOLOGY "Measuring acid and alkaline phosphatase activities" page 328, no. 20 379h; & JP-A-59 143 600 (JAPAN KOKAI TOKKYO KOHO) -- | 1,14 | **TECHNICAL FIELDS SEARCHED (Int. Cl.6)**<br><br>C 12 Q<br>G 01 N |
| A | CHEMICAL ABSTRACTS, vol. 100, no. 25, 1984, June 18, Columbus, Ohio, USA F. MIZUTANI et al. "Amperometric determination of acid | 1,14 | |

The present search report has been drawn up for all claims

| Place of search | Date of completion of the search | Examiner |
|---|---|---|
| VIENNA | 15-12-1995 | SCHNASS |

European Patent Office

# EUROPEAN SEARCH REPORT

Application Number

-2-
EP 95202634.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 6) |
|---|---|---|---|
| | phosphatase with an immobilized enzyme electrode"<br>page 226, no. 205 389p;<br>& Anal. Chem. Symp. Ser.<br>1983, 17(Chem.Sens.), 644-7<br>---- | | |
| | | | TECHNICAL FIELDS SEARCHED (Int. Cl. 6) |

The present search report has been drawn up for all claims

| Place of search | Date of completion of the search | Examiner |
|---|---|---|
| VIENNA | 15-12-1995 | SCHNASS |

EPO FORM 1503 03.82 (P0401)